# FASCICULE DE BREVET EUROPEEN

(11) **EP 0 970 692 B1**
(45) Date de publication et mention de la délivrance du brevet: **22.10.2003**
(21) Numéro de dépôt: 99401657.4
(22) Date de dépôt: 02.07.1999
(51) Int. Cl.: A61K 7/48, A61K 7/06

(54) **Nouvelles compositions cosmétiques comprenant un polymère filmogène**
Neue Zusammensetzungen enthaltend ein filmbildendes Polymer
Novel cosmetic compositions containing a filmforming polymer

(30) Priorité: 07.07.1998 FR 9808687
(43) Date de publication de la demande: 12.01.2000
(73) Titulaire: L'OREAL, 75008 Paris (FR)
(72) Inventeur: Tournilhac, Florence, 75011 Paris (FR); Lemann, Patricia, 94000 Créteil (FR)
(74) Mandataire: Kromer, Christophe

(56) Documents cités:
- EP-A- 0 555 155
- WO-A-98/00495
- WO-A-98/00499
- US-A- 4 155 892

## Description

La présente invention se rapporte à une composition du type émulsion, de consistance visqueuse destinée au soin, au traitement ou au maquillage de la peau aussi bien du visage que du corps humain, des fibres kératiniques comme les cils, les sourcils, les cheveux ou encore des lèvres.

Cette composition peut être un fond de teint, un fard à joues ou à paupières, un anticemes, une crème à lèvres, un mascara, un produit de maquillage du corps, lorsqu'elle se présente sous forme colorée ou bien être une crème de soin pour la peau, un après-shampooing, un shampooing, une crème de protection solaire ou de coloration de la peau ou encore une pommade dermatologique, lorsqu'elle se présente sous forme non colorée.

Les compositions de soin ou de maquillage épaissies contiennent généralement un agent épaississant facilitant la prise de produit hors du conditionnement, sans perte de produit, permettant de répartir de façon homogène le produit sur la zone à traiter ou de prélever les quantités suffisantes de produit, pour obtenir l'effet cosmétique recherché. Selon ce type de composition, l'agent épaississant peut être hydrophile ou lipophile. Lorsque la composition est suffisamment épaisse, une prise de produit au doigt fait apparaître, à la surface du produit dans le conditionnement, des parties en creux et lors de la prise suivante, la surface de produit est restée en l'état, notamment lors de la fermeture précédente du pot; la crème paraît alors polluée, provoquant une insatisfaction de la part des consommateurs.

Le non nivellement en surface de la crème, après chaque utilisation, apparaît en particulier pour une crème de texture riche et/ou épaissie, ne s'écoulant pas.

Il subsiste donc le besoin d'une composition de maquillage, de traitement ou de soin de la peau et des fibres kératiniques présentant un bon nivellement de la surface dans son conditionnement, donnant l'impression d'ouvrir à chaque fois un nouveau pot, et de toucher un produit non pollué.

La demande EP-A-555155 décrit une composition cosmétique détergeante contenant un agent tensioactif non ionique alkylpolyglycoside ou polyglycérolé et un uréthane-polyéther ayant des propriétés démêlante des cheveux.

Les demandes WO 98/00495 et WO 9800499 décrivent des compositions nettoyantes comprenant des tensioactifs hydrosolubles et un épaississant polymère associatif, notamment de type polyuréthane.

Le brevet US 4155892 décrit des compositions aqueuses comprenant un épaississant polyuréthanes et des tensioactifs.

Aussi, la présente invention a pour objet une composition contenant un milieu physiologiquement acceptable, épaissie par un polyuréthanne associatif, ce milieu contenant une phase grasse liquide et une phase aqueuse en émulsion, la composition étant une émulsion huile-dans-eau et ne contenant pas de tensioactif.

L'invention a encore pour objet l'utilisation d'un polyuréthanne associatif dans une composition de type huile-dans-eau de consistance visqueuse et ne contenant pas de tensioactif.

L'invention a également pour objet l'utilisation d'un polyuréthanne associatif pour la fabrication d'une composition dermatologique de type huile-dans-eau de consistance visqueuse et ne contenant pas de tensioactif.

L'invention a aussi pour objet l'utilisation d'un polyuréthanne associatif dans ou pour la fabrication d'une composition de type émulsion huile-dans-eau ne contenant pas de tensioactif, pour assurer, lors de l'application sur la peau, une autocicatrisation et/ou un nivellement du relief de la peau.

Grâce au polyuréthanne associatif, on obtient une composition de texture épaisse, s'étalant bien, rhéofluidifiable (dont la viscosité diminue avec la vitesse de cisaillement), de viscoélasticité remarquable.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple: copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquence hydrophiles sont liées par d'autres liaisons chimiques aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010 et le Sérad 1035 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS.

Les polymères utilisables dans l'invention sont en particulier ceux décrits dans l'article de G. Fonnum, J. Bakke et Fk. Hansen - Colloid Polym. Sci 271, 380.389 (1993).

La composition selon l'invention contient un ou plusieurs polyuréthannes associatifs, en une quantité suffisante pour obtenir une composition épaissie rhéofluidifiante, de viscosité de 1 000 à 10 000 cp (soit 1 à 10 Pa.s), de préférence 2 000 à 6 000 cp (soit 2 à 6 Pa.s) mesurée à 25°C avec un mobile tournant à 100 s⁻¹ et par exemple avec un viscosimètre Rhéomat 115. En vue d'obtenir une composition épaissie stable, il est avantageux d'utiliser au moins 5 mg/m² de polyuréthanne associatif par surface développée d'huile dans eau, et mieux au moins 10 mg/m².

La composition selon l'invention est à phase continue aqueuse. Elle peut être une émulsion simple huile-dans-eau. Elle se présente en particulier sous la forme d'une crème ou d'une pommade.

Les compositions à phase continue ou externe aqueuse présentent t'avantage de s'étaler facilement, d'être légère, de bien pénétrer dans la peau, d'être non collante et d'apporter de la fraîcheur à la peau lors de l'application, contrairement à des compositions à phase continue huileuse.

Selon l'invention, la phase aqueuse peut contenir de l'eau, un milieu hydroalcoolique et notamment un milieu contenant des polyols.

Avantageusement, le ou les polyuréthannes associatifs jouent le rôle d'émulsionnant de la phase huileuse dans la phase aqueuse. Lorsque le polyuréthanne associatif est utilisé comme seul émulsionnant, il est très avantageusement présent en quantité allant de 2 à 20 % du poids total de la composition, de préférence en une quantité allant de 3 à 10 %.

La composition ne comporte pas de tensioactif, ce qui est particulièrement intéressant pour les personnes à peau sensible. En particulier, la composition peut être utilisée comme excipient pour une pommade à propriété dermatologique.

Lorsque l'émulsion est du type huile-dans-eau, le polyuréthanne associatif permet l'obtention d'un « réseau » polymérique sur la peau, lors de l'application, assurant notamment l'autocicatrisation et le nivellement du relief de la peau.

La composition selon l'invention peut contenir avantageusement plusieurs matières colorantes comme les colorants lipophiles ou hydrophiles, les pigments, et les nacres. Par pigment il faut comprendre des particules blanches ou colorées, minérales ou organiques, insolubles dans la phase grasse liquide, destinées à colorer et/ou opacifier la composition. Par nacres, il faut comprendre des particules irisées, notamment produites par certains mollusques dans leur coquille ou bien synthétisées. Par colorants, il faut comprendre des composés généralement organiques solubles dans les corps gras comme les huiles ou dans une phase hydroalcoolique.

Les matières colorantes peuvent représenter de 0,01 à 60 % du poids total de la composition, de préférence de 0, 05 à 35 % et plus particulièrement de 1 à 20 %.

Comme pigments minéraux utilisables dans l'invention, on peut citer les oxydes de titane, de zirconium ou de cérium ainsi que les oxydes de zinc, de fer ou de chrome et le bleu ferrique, leurs mélanges. Parmi les pigments organiques utilisables dans l'invention, on peut citer le noir de carbone, et les laques de baryum, strontium, calcium, aluminium, leurs mélanges.

Les colorants liposolubles utilisables dans l'invention sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine, leurs mélanges. Ils peuvent représenter de 0,01 à 20 % du poids total de la seconde composition et mieux de 0,1 à 10 %. Les colorants hydrosolubles utilisables sont notamment le sulfate de cuivre, de fer, des sulfopolyesters hydrosolubles tels que ceux décrits dans les documents FR-96 154152, les rhodamines, les colorants naturels (carotène, jus de betterave), bleu de méthylène, leurs mélanges.

Les nacres peuvent être présentes dans la composition de l'invention à raison de 0 à 20 % du poids total de la composition, de préférence à un taux de l'ordre de 1 à 15 %. Parmi les nacres utilisables dans la composition, on peut citer le mica recouvert d'oxyde de titane, d'oxyde de fer, de pigment naturel ou d'oxychlorure de bismuth tel que le mica titane coloré, leurs mélanges.

La composition selon l'invention comprend une phase huileuse, c'est-à-dire une phase grasse liquide à température ambiante (25°C), contenant une ou plusieurs huiles liquides compatibles entre elles. Les huiles selon l'inventeur ne sont pas des tensioactifs et présentent avantageusement une valeur d'lOB (Balance Inorganique/Organique) inférieure à 0,42. Le paramètre de l'IOB est connu de l'homme du métier à partir d'un certain nombre de publications comme l'article de A. FUJITA Pharm. Bull 2, 163-173 (1954) et les documents JO 9/151109, J08/217639 de Shiseïdo ou J09/175925 de Kosé.

Comme huile utilisable dans la composition de l'invention, on peut citer :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le partéam ; les isoparaffines comme l'isohexadécane et l'isodécane ;
- les esters et les éthers de synthèse notamment d'acides gras comme les huiles de formule R₁COOR₂ dans laquelle R₁ représente le reste d'un acide gras supérieur comportant de 7 à 29 atomes de carbone et R₂ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone en particulier ramifié comme l'octyldodécanol, le 2-butyloctanol, le 2-hexyldécanol, le 2-undécylpentadécanol ;
- les huiles fluorées partiellement hydrocarbonées et/ou siliconées comme celle décrites dans le document JP-A-2-295912 ;
- les huiles siliconées comme les polyméthylsiloxanes (PDMS) volatiles ou non, linéaires ou cycliques, liquides ou pâteux à température ambiante ; les silicones phénylées comme les phényl triméthicones, les diphényl diméthicones, les phényl diméthicones, les phényltriméthylsiloxy diphényl siloxanes ;
- les huiles fluorées et les huiles fluorosiliconées ;
- leurs mélanges.

La phase huileuse peut représenter de 0,1 à 30 % du poids total de la composition et mieux de 10 à 25 %.

Selon l'invention, la composition peut, en outre, comprendre tout ingrédient additionnel classiquement utilisé dans les domaines cosmétiques et dermatologiques.

Comme ingrédient additionnel utilisable dans l'invention, on peut citer, les charges, les conservateurs, les antioxydants, les parfums, les actifs cosmétiques ou dermatologiques, leurs mélanges. Les quantités de ces différents ingrédients, sont celles généralement utilisées par exemple à des quantités allant de 0,01 à 30 % du poids total de la composition. La nature de ces ingrédients et leurs proportions doivent être compatibles avec l'obtention des propriétés recherchées pour la composition de l'invention, comme les propriétés rhéofluidifiantes, de stabilité, de viscoélasticité.

Par charges, il faut comprendre des particules incolores ou blanches, minérales ou de synthèse, lamellaires ou non lamellaires. Ces charges peuvent être introduites dans la composition en vue de notamment d'en modifier sa texture. Elles peuvent être présentes à raison de 0 à 35 % du poids total de la chaque composition, de préférence 0,5 à 15 %. On peut notamment citer le talc, le stéarate de zinc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple) ou encore la silice.

Selon la présence ou non de matière colorante et/ou d'actif cosmétique ou dermatologique, la composition de l'invention peut se présenter sous la forme de crème de soin de la peau, y compris du cuir chevelu ou des cheveux, des ongles, de protection solaire ou de coloration de la peau, de fond de teint, de produit à lèvres comme les gloss, en terminologie anglo-saxonne, d'eye liner, mascara, de produit anti-cernes, de produit de maquillage du corps, d'après-shampooing, de crème de traitement dermatologique de la peau y compris le cuir chevelu.

Les compositions de l'invention peuvent être obtenues de façon classiques, par mélange des ingrédients hydrophiles dans la phase aqueuse, mélange des ingrédients lipophiles dans la phase huileuse, le ou les polyuréthannes associatifs et les tensioactifs étant en particulier mélangés à la phase aqueuse, puis par introduction de la phase huileuse dans la phase aqueuse et enfin mélange de façon à obtenir un produit homogène.

La description qui suit est donnée à titre illustratif et sans caractère limitatif. Les quantités des différents constituants sont donnés en % en poids.

### Exemple 1 de fond de teint rhéofluidifiant

- Huile d'amandes d'abricots 13 %
- Sicovit jaune 10 E 172 0,8 %
- Sicomet Brun ZP 3569 0,67 %
- Sicovit Noir 85 E 172 0,23 %
- Hombitan anatase FF Pharma 5, 3 %
- Paraoxybenzoate de propyle 0,1 %
- Paraoxybenzoate de méthyle 0,2 %
- Triéthanolamine à 99 % minimum 0,15 %
- Polyuréthanne associatif (Ser-Ad FX 1 100) 2,24 %
- Cyclohexasiloxane (Dow Coming 246 fluid) 10 %
- Glycérine 7 %
- Eau déminéralisée stérilisée qps 100 %
- Stéarate de polyéthylène glycol (PEG 8) 1,3 %
- Acide stéarique 0,3 %
- Alcool stéarylique 0,5 %
- Stéarate de glucose 1,3%
Cette formule présente une viscosité qui diminue avec la vitesse de cisaillement, mesurée à 25°C avec un Rhéomat 115 : 2 x 10⁵cp à 0,3 s⁻¹ ; 4 x 10⁴cp à 10s⁻¹ ; 5 x 10³cp à 100s⁻¹ ; 2 x 10² cp à 2 x 10³s⁻¹.

Cette formule appliquée sur la peau conduit à un film lisse, légèrement satiné. En outre, après prise au doigt du produit dans le pot (ou cisaillement manuel), la surface du produit dans le pot reprend sa forme initiale (cicatrisation de la surface).

### Exemple 2 de crème de soin rhéofluidifiante

- Huile d'amandes d'abricots 13 %
- Paraoxybenzoate de propyle 0,1 %
- Paraoxybenzoate de méthyle 0,2 %
- Polyuréthanne associatif (Ser-Ad FX 1 100) 2,24 %
- Cyclohexasiloxane (Dow Corning 246 fluid) 10 %
- Glycérine 7 %
- Eau déminéralisée stérilisée qps 100 %
Cette émulsion, sans tensioactif additionnel, présente une viscosité de 5 x 10³ cp à 10 s⁻¹ ; 3 x 10³ cp à 100 s⁻¹ ; 2.10²cp à 2 x 10³s⁻¹, mesurée à 25°C avec un viscositmètre Rhéomat 115.

Les compositions précédentes sont préparées de la façon suivante : on prépare séparément les phases grasse et aqueuse que l'on chauffe autour de 70°-80°C. On introduit la phase huileuse dans la phase aqueuse sous agitation avec un agitateur mécanique du type Moritz. Lorsque l'émulsion ainsi formée commence à atteindre les 40°C, on ajoute progressivement le gel de polyuréthane préalablement préparé dans l'eau (concentration du prégel 5%). On termine l'agitation avec un agitateur mécanique du type Rainery.

## Revendications

1. Composition contenant un milieu physiologiquement acceptable, épaissie par un polyuréthanne associatif, le milieu contenant une phase grasse liquide et une phase aqueuse en émulsion, la composition étant une émulsion huile-dans-eau et ne contenant pas de tensioactif.

2. Composition selon la revendication 1, dans laquelle le polyuréthanne associatif est présente à au moins 5 mg par m² de surface déployée d'huile dans l'eau.

3. Composition selon la revendication 1 ou 2, dans laquelle le polyuréthanne associatif est présent à au moins 10 mg/m² de surface déployée d'huile dans eau.

4. Composition selon l'une des revendications précédentes, dans laquelle le polyuréthanne associatif contient de 2 à 20 % du poids total de la composition de polyuréthanne associatif.

5. Composition selon l'une des revendications précédentes, dans laquelle le polyuréthanne associatif représente de 3 à 10 % du poids total de la composition.

6. Composition selon l'une des revendications précédentes, dans laquelle le polyuréthanne associatif est un polymère séquencé ou greffé comportant au moins deux chaînes alkyles ayant de C₆ à C₃₀ d'atomes de carbone, séparées par une séquence hydrophile.

7. Composition selon l'une des revendications précédentes, dans laquelle le polyuréthanne associatif comporte une séquence hydrophile polyoxyéthylénée.

8. Composition selon l'une des revendications précédentes, dans laquelle le polyuréthanne associatif est un polymère tribloc.

9. Composition selon l'une des revendications précédentes, présentant une viscosité de 1 000 à 10 000 cp (1 à 10 Pa.s), de préférence 2 000 à 6 000 cp (2 à 6 Pa.s) mesurée à 100 s⁻¹ à 25°C.

10. Composition selon l'une des revendications précédentes, dans laquelle la phase grasse liquide représente de 0,1 à 30 % du poids total de la composition.

11. Composition selon l'une des revendications précédentes, dans laquelle la phase grasse liquide représente de 10 à 25 % du poids total de la composition.

12. Composition selon l'une des revendications précédentes, dans laquelle la phase grasse liquide comprend un huile ou un mélange d'huiles choisies parmi les huiles d'origine minérale, végétale, animale, synthétique, hydrocarbonée, siliconée, et/ou fluorée et leurs mélanges ayant de préférence un IOB inférieur à 0,42.

13. Composition selon l'une des revendications précédentes, dans laquelle le polyuréthanne associatif assure l'émulsion de l'huile dans l'eau.

14. Composition selon l'une des revendications précédentes, contenant au moins une matière colorante choisie parmi les colorants solubles, les colorants hydrosolubles, les pigments et les mélanges.

15. Composition selon l'une des revendications précédentes, contenant au moins un ingrédient choisi parmi les conservateurs, les charges, les parfums, les actifs cosmétiques, les actifs dermatologiques, les antioxydants et leurs mélanges.

16. Composition selon l'une des revendications précédentes, se présentant sous forme de crème de soin, de crème solaire ou de coloration de la peau, de fond de teint, produit à lèvres, eye-liner, mascara, crème de soin pour les cheveux et les ongles, produit de maquillage du corps, d'après-shampooing, de produit anticemes.

17. Utilisation d'un polyuréthanne associatif dans une composition de type huile-dans-eau de consistance visqueuse et ne contenant pas de tensioactif.

18. Utilisation d'un polyuréthanne associatif pour la fabrication d'une composition dermatologique de type huile-dans-eau de consistance visqueuse et ne contenant pas de tensioactif.

19. Utilisation d'un polyuréthanne associatif dans ou pour la fabrication .d'une composition de type émulsion huile-dans-eau ne contenant pas de tensioactif, pour assurer, lors de l'application sur la peau, une autocicatrisation et/où un nivellement du relief de la peau.

## Patentansprüche

1. Zusammensetzung, die ein physiologisch akzeptables Medium enthält und mit einem assoziativen Polymer verdickt ist, wobei das Medium eine flüssige Fettphase und eine wässrige Phase als Emulsion enthält und wobei die Zusammensetzung eine Öl-in-Wasser-Emulsion ist und keinen grenzflächenaktiven Stoff enthält.

2. Zusammensetzung nach Anspruch 1, wobei das assoziative Polyurethan in einer Menge von mindestens 5 mg pro m² der gebildeten Oberfläche des Öls in Wasser vorliegt.

3. Zusammensetzung nach Anspruch 1 oder 2, wobei das assoziative Polyurethan in einer Menge von mindestens 10 mg pro m² der Oberfläche des Öls in Wasser vorliegt.

4. Zusammensetzung nach einem der vorhergehenden Ansprüche, wobei das assoziative Polyurethan 2 bis 20 Gew.-% des Gesamtgewichts der Zusammensetzung ausmacht.

5. Zusammensetzung nach einem der vorhergehenden Ansprüche, wobei das assoziative Polyurethan 3 bis 10 Gew.-% des Gesamtgewichts der Zusammensetzung ausmacht.

6. Zusammensetzung nach einem der vorhergehenden Ansprüche, wobei das assoziative Polyurethan ein sequenzielles Polymer oder Pfropfpolymer ist, das mindestens zwei Alkylgruppen mit 6 bis 30 Kohlenstoffatomen enthält, die über eine hydrophile Sequenz voneinander getrennt sind.

7. Zusammensetzung nach einem der vorhergehenden Ansprüche, wobei das assoziative Polyurethan eine hydrophile polyethoxylierte Sequenz enthält.

8. Zusammensetzung nach einem der vorhergehenden Ansprüche, wobei das assoziative Polyurethan ein Dreiblock-Polymer ist.

9. Zusammensetzung nach einem der vorhergehenden Ansprüche, die eine Viskosität von 1 000 bis 10 000 cP (1 bis 10 Pa·s) und vorzugsweise 2 000 bis 6 000 cP (2 bis 6 Pa·s) aufweist, die bei 25 °C und 100 s⁻¹ gemessen wird.

10. Zusammensetzung nach einem der vorhergehenden Ansprüche, wobei die flüssige Fettphase 0,1 bis 30 % des Gesamtgewichts der Zusammensetzung ausmacht.

11. Zusammensetzung nach einem der vorhergehenden Ansprüche, wobei die flüssige Fettphase 10 bis 25 % des Gesamtgewichts der Zusammensetzung ausmacht.

12. Zusammensetzung nach einem der vorhergehenden Ansprüche, wobei die flüssige Fettphase ein Öl oder ein Gemisch von Ölen enthält, die unter den Ölen mineralischer, pflanzlicher, tierischer oder synthetischer Herkunft, Kohlenwasserstoffölen, Siliconölen und/oder fluorierten Ölen und deren Gemischen ausgewählt ist, wobei sie vorzugsweise einen IOB-Wert unter 0,42 besitzen.

13. Zusammensetzung nach einem der vorhergehenden Ansprüche, wobei das assoziative Polyurethan das Emulgieren des Öls in dem Wasser gewährleistet.

14. Zusammensetzung nach einem der vorhergehenden Ansprüche, die mindestens ein Farbmittel enthält, das unter den löslichen Farbstoffen, wasserlöslichen Farbstoffen, Pigmenten und deren Gemischen ausgewählt ist.

15. Zusammensetzung nach einem der vorhergehenden Ansprüche, die mindestens einen Bestandteil enthält, der unter den Konservierungsmitteln, Füllstoffen, Parfums, kosmetischen Wirkstoffen, dermatologischen Wirkstoffen, Antioxidantien und deren Gemischen ausgewählt ist.

16. Zusammensetzung nach einem der vorhergehenden Ansprüche, die als Creme zur Pflege, Creme zum Lichtschutz oder zum Färben der Haut, Make-up, Produkt für die Lippen, Eyeliner, Macara, Creme zur Pflege der Haare und für die Nägel, Produkt zum Schminken des Körpers, Haarpflegemittel, das nach der Haarwäsche angewandt wird, oder Produkt gegen Augenringe vorliegt.

17. Verwendung eines assoziativen Polyurethans in einer Zusammensetzung von viskoser Konsistenz vom Öl-in-Wasser-Typ, die keine grenzflächenaktiven Stoffe enthält.

18. Verwendung eines assoziativen Polyurethans zur Herstellung einer dermatologischen Zusammensetzung von viskoser Konsistenz vom Öl-in-Wasser-Typ, die keine grenzflächenaktiven Stoffe enthält.

19. Verwendung eines assoziativen Polyurethans in einer Zusammensetzung oder zur Herstellung einer Zusammensetzung vom Öl-in-Wasser-Typ, die keine grenzflächenaktiven Stoffe enthält, um beim Auftragen auf die Haut eine Selbstregenerierung und/oder eine Einebnung des Hautreliefs zu gewährleisten.

## Claims

1. Composition containing a physiologically acceptable medium thickened with an associative polyurethane, the medium containing a liquid oily phase and an aqueous phase in the form of an emulsion, the composition being an oil-in-water emulsion and containing no surfactant.

2. Composition according to Claim 1, in which the associative polyurethane is present in an amount of at least 5 mg per m² of deployed surface of oil in water.

3. Composition according to Claim 1 or Claim 2, in which the associative polyurethane is present in amount of at least 10 mg/m² of deployed surface of oil in water.

4. Composition according to any one of the preceding Claims, in which the associative polyurethane contains 2% to 20% of the total weight of the associative polyurethane composition.

5. Composition according to one of the preceding claims, in which the associative polyurethane represents 3% to 10% of the total composition weight.

6. Composition according to one of the preceding claims, in which the associative polyurethane is a block or graft polymer comprising at least two alkyl chains containing C₆ to C₃₀ carbon atoms, separated by a hydrophilic block.

7. Composition according to one of the preceding Claims, in which the associative polyurethane comprises a polyoxyethylenated hydrophilic block.

8. Composition according to one of . the preceding Claims, in which the associative polyurethane is a triblock polymer.

9. Composition according . to one of the preceding Claims, having a viscosity of 1000 to 10000 cp (1 to 10 Pa.s), preferably 2000 to 6000 cp (2 to 6 Pa.s), measured at 100 s⁻¹ at 25°C.

10. Composition according to one of the preceding Claims, in which the liquid oily phase represents 0.1% to 30% of the total composition weight.

11. Composition according to one of the preceding Claims, in which the. liquid oily phase represents 10% to 25% of the total composition weight.

12. Composition according to one of the preceding Claims, in which the liquid oily phase comprises an oil or a mixture of oils selected from oils of mineral, vegetable, animal, synthetic, hydrocarbon, silicone and/or fluorinated origin and mixtures thereof, preferably having an IOB of less than 0.42.

13. Composition according to one of the preceding Claims, in which the associative polyurethane ensures emulsification of the oil in water.

14. Composition according to one of the preceding Claims, containing at least one colouring material selected from soluble colorants, hydrosoluble colorants, pigments and mixtures thereof.

15. Composition according to one of the preceding Claims, containing at least one ingredient selected from preservatives, fillers, fragrances, active cosmetic ingredient, active dermatological ingredients, antioxidants and mixtures thereof.

16. Composition according to one of the preceding Claims, in the form of a skin care cream, sun cream for the skin or skin colouring cream, foundation, lip product, eyeliner, mascara, hair care and nail care cream, body makeup product, conditioner or concealer.

17. Use of an associative polyurethane in an oil-in-water type composition with a viscous consistency containing no surfactant.

18. Use of an associative polyurethane for the production of a dermatological oil-in-water type composition with a viscous consistency and containing no surfactant.

19. Use of an associative polyurethane in or for the production of an oil-in-water emulsion type composition containing no surfactant, to provide complete coverage and/or levelling of the skin profile on application to the skin.
